# EUROPEAN PATENT APPLICATION

(11) **EP 4 711 468 A1**
(43) Date of publication of application: **18.03.2026**
(21) Application number: 24832466.7
(22) Date of filing: 27.06.2024
(51) Int. Cl.: C12P 13/08, C07C 227/42, C07C 229/08

(54) **METHOD FOR PREPARING L-VALINE CRYSTALS AND/OR GRANULES BY USING PH CONTROL**

(30) Priority: 28.06.2023 KR 20230083410
(71) Applicant: CJ Cheiljedang Corporation, Seoul 04560 (KR)
(72) Inventor: KANG, Seung Hoon, Seoul 04560 (KR); JEONG, Jae Yun, Seoul 04560 (KR); KIM, Jun-Woo, Seoul 04560 (KR); SHIN, Ji Hyun, Seoul 04560 (KR); KIM, Sumin, Seoul 04560 (KR); KANG, Ji-hun, Seoul 04560 (KR)
(74) Representative: Meissner Bolte Partnerschaft mbB
(86) International application number: PCT/KR2024/008993
(87) International publication number: WO 2025/005689

(57) **Abstract**

The present disclosure relates to a method for producing valine crystals and/or valine granules by using pH adjustment.

## Description

### [Technical Field]

The present disclosure relates to a method for producing valine crystals and granules, which can reduce the amount of steam required during the production of valine granules and improve the production efficiency of L-valine granules by forming valine crystals and mixed granules having a low moisture content.

### [Background Art]

In order to obtain highly purified amino acids through fermentation, it is essential to perform a step of separation or purification of amino acids to remove byproducts after a fermentation process.

However, with regard to amino acid products for feed, products with a high amino acid content (over 98%) are not necessary, and it may be preferred to produce granular products with a high content (over 70%) while including other nutritionally valuable components contained in fermentation broth without generating waste during purification.

In the existing art (US 7514111 B2), to produce amino acid granular products for feed additives, fluidized bed granulators are used after evaporating a large amount of moisture in fermentation broth. In the case of amino acids with high solubility, crystals are not produced even when the moisture is evaporated until the solid content reaches about 60 to 65% (a moisture content of 35 to 40%). Therefore, granulation can be achieved by the fluidized bed granulation method in which the fermentation broth is sprayed through a nozzle. However, in the case of valine, which has low solubility, crystals are produced even when the solid content is as low as in a range of about 12 to 15% (a moisture content of 85 to 88%), thus, it is not appropriate to reduce the moisture content of fermentation broth to prevent crystal formation. Therefore, a large amount of moisture must be evaporated during the drying process, resulting in a large consumption of energy during drying.

### [Prior Art Documents]

### [Patent Documents]

(Patent Document 1) US 7514111 B2
(Patent Document 2) US 10072278 B2

### [Disclosure]

### [Technical Problem]

One object of the present disclosure is to provide a method for producing valine crystals, comprising: preparing a fermentation broth comprising valine; adjusting the pH of the fermentation broth; concentrating the fermentation broth to prepare a concentrated fermentation broth; and separating valine crystals comprising moisture from the concentrated fermentation broth.

Another object of the present disclosure is to provide a method for producing valine granules, comprising: preparing a fermentation broth comprising valine; adjusting the pH of the fermentation broth; concentrating the fermentation broth to prepare a concentrated fermentation broth; separating valine crystals comprising moisture from the concentrated fermentation broth; mixing the valine crystals with a seed to produce mixed granules of valine; and drying the mixed granules of valine.

### [Technical Solution]

One aspect of the present disclosure provides a method for producing valine crystals, comprising: preparing a fermentation broth comprising valine; adjusting the pH of the fermentation broth; concentrating the fermentation broth to prepare a concentrated fermentation broth; and separating valine crystals comprising moisture from the concentrated fermentation broth.

Another aspect of the present disclosure provides a method for producing valine granules, comprising: preparing a fermentation broth comprising valine; adjusting the pH of the fermentation broth; concentrating the fermentation broth to prepare a concentrated fermentation broth; separating valine crystals comprising moisture from the concentrated fermentation broth; mixing the valine crystals with a seed to produce mixed granules of valine; and drying the mixed granules of valine.

### [Advantageous Effects]

According to the present disclosure, mixed granules of valine having a large size and a low moisture content can be produced by concentrating the fermentation broth comprising valine, separating valine wet crystals, and mixing the separated valine wet crystals with a seed. The mixed granules of valine having a low moisture content have the advantage that less energy is required for drying. In particular, according to the present disclosure, the moisture content in the mixed granules of valine can be further reduced by adjusting the pH of the fermentation broth and/or controlling the concentration rate.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description below.

Additionally, those of ordinary skill in the art may be able to recognize or confirm, using only conventional experimentation, many equivalents to the particular aspects of the invention described herein. Furthermore, it is also intended that these equivalents be included in the present disclosure.

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" includes "and/or".

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

The present disclosure relates to a method for producing valine granules and a production device thereof. In the present disclosure, mixed granules of valine comprising a mixture of valine wet crystals and a seed are formed and dried to reduce the amount of steam consumed in the production process of valine, which is an amino acid with low solubility, thereby increasing the production efficiency of valine granules.

The method for producing L-valine crystals according to the present disclosure comprises: preparing a fermentation broth comprising valine; adjusting the pH of the fermentation broth; concentrating the fermentation broth to prepare a concentrated fermentation broth; and separating valine crystals comprising moisture from the concentrated fermentation broth. Hereinafter, each step will be described in detail.

The step of preparing a fermentation broth is a step of preparing a fermentation broth comprising valine. Valine comprised in the fermentation broth is an amino acid with low solubility in water. Herein, low solubility does not mean that the solubility is below a certain standard. The fermentation broth prepared in the step of preparing a fermentation broth may be a fermentation product prepared by metabolism of a strain. As used herein, the term "fermentation product" may refer to a product resulting from enzymatic or metabolic decomposition of organic substances using microorganisms, *e.g.,* microorganisms for producing valine. For example, the fermentation product may include a culture product itself obtained by culturing a microorganism in a culture medium, or a concentrate, dried product, or freeze-dried product of the culture product obtained by removing the strain therefrom. In addition, in this case, the fermentation broth may comprise the entire fermentation product comprising valine, or may be one in which impurities have been removed from the fermentation product comprising valine.

The fermentation broth may comprise valine as a main component, but may further comprise other components. For example, in addition to valine, other amino acids, organic acids, and inorganic substances may be further provided in the fermentation broth and these may be dissolved in water. The concentration of valine in the fermentation product may be about 1 g/L to about 200 g/L. However, the concentration of valine described above is merely exemplary, and a fermentation broth having a concentration outside the above-described range may also be used in the method for producing valine crystals according to one embodiment of the present disclosure.

The "microorganism for producing valine" or "microorganism having valine-producing ability" used in the step of preparing the fermentation broth of the present disclosure may include all wild-type microorganisms naturally having valine-producing ability, or microorganisms having natural or artificial genetic modifications, in which valine-producing ability has been imparted to a parent strain having no valine-producing ability, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired amino acid, *i.e.,* valine. Specifically, as used herein, the microorganism for producing valine or the microorganism having valine-producing ability may be a microorganism in which some genes in the biosynthesis pathway of valine are enhanced or weakened, or some genes in the degradation pathway of valine are enhanced or weakened. The "enhancement" or "increase" in the valine-producing ability of the microorganism of the present disclosure may mean that the valine-producing ability of the microorganism of the present disclosure is enhanced compared to that of a microorganism other than the microorganism of the present disclosure, a parent strain, or a non-modified microorganism. In one example, the microorganism of the present disclosure may have an enhanced valine producing ability by about 1% or more, 10% or more, 100% or more, 200% or more, 500% or more, 1000% or more, 1100% or more, 1200% or more, 1300% or more, or about 1.01 times or more, 2 times or more, 5 times or more, 10 times or more, 11 times or more, 12 times or more, or 13 times or more compared to the valine-producing ability of other microorganisms, but is not limited thereto. As used herein, the term "about" refers to a range including all of ±0.5, ±0.4, ±0.3, ±0.2, ±0.1, *etc.,* and it includes all values equivalent to those which come immediately after the term "about" or those in a similar range, but is not limited thereto.

The above-mentioned microorganism used in the step of preparing the fermentation broth of the present disclosure may be at least one selected from the group consisting of *Candida famata,* which is a yeast, *Eremothecium ashbyii* and *Ashbya gossypii,* which are ascomycetes, *Bacillus subtilis,* and microorganisms belonging to the genus *Corynebacterium,* which are bacteria.

For example, when the microorganism used in the step of preparing the fermentation broth of the present disclosure is a microorganism belonging to the genus *Corynebacterium,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* more specifically, *Corynebacterium glutamicum,* but is not limited thereto.

The microorganism of the genus *Corynebacterium,* especially *Corynebacterium glutamicum,* is a Gram-positive microorganism that is widely used in the production of L-amino acids and other beneficial substances. In order to produce L-amino acids and other beneficial substances, various research has been performed to develop microorganisms with high-efficiency production and technologies for fermentation processes. For example, target-specific approaches, such as a method of increasing expression of a gene encoding an enzyme involved in L-valine biosynthesis or a method of removing a gene unnecessary for biosynthesis, have been widely used. In the present disclosure, a fermentation broth comprising amino acids can be prepared using a strain of the genus *Corynebacterium.*

According to one embodiment of the present disclosure, *Corynebacterium glutamicum* used to prepare the fermentation broth comprising valine may be an L-valine biosynthesis overexpressing strain in which the L-valine biosynthesis regulatory region is inactivated by transforming *Corynebacterium glutamicum* ATCC13032, but is not limited thereto. Alternatively, the *Corynebacterium glutamicum* may be a strain transformed with pDZDvalL, pDZDvalP, or pDZDvalS vectors using the chromosomes of *Corynebacterium glutamicum* KCCM11201P, *Corynebacterium glutamicum* KCCM11336P, *Corynebacterium glutamicum* KCCM11337P, *Corynebacterium glutamicum* KCCM11338P, and *Corynebacterium glutamicum* KCCM11201P_DvaIA as a template. In another embodiment, the *Corynebacterium glutamicum* may be a strain of *Corynebacterium glutamicum* ATCC13032_DvaIL, *Corynebacterium glutamicum* ATCC13032_DvalP, or *Corynebacterium glutamicum* ATCC13032_DvalS, but is not limited thereto, and may include all microorganisms capable of producing a fermentation broth comprising valine without limitation (US 10072278 B2).

The step of preparing the fermentation broth may further comprise a step of culturing "a microorganism for producing valine". The cultivation of microorganisms may be performed in a suitable culture medium and culture conditions known in the art. Such a cultivation process may be easily adjusted for use by those skilled in the art according to the microorganism to be selected. Specifically, the cultivation may be a batch culture, a continuous culture, and a fed-batch culture, but is not limited thereto.

As used herein, the term "medium" refers to a mixture of materials that comprises nutrient materials required for the cultivation of the microorganism of the present disclosure as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

Next, the step of adjusting the pH of the fermentation broth is performed. In particular, the pH of the fermentation broth may be adjusted to be about greater than 3.0 to less than 6.0. In some cases, the pH of the fermentation broth may be adjusted to about 3.5 to less than 6.0, about 4.0 to less than 6.0, about 4.5 to less than 6.0, about 5.0 to less than 6.0, about 5.5 to less than 6.0, about greater than 3.0 to 5.5, about 3.5 to 5.5, about 4.0 to 5.5, about 4.5 to 5.5, about 5.0 to 5.5, about greater than 3.0 to 5.0, about 3.5 to 5.0, about 4.0 to 5.0, about 4.5 to 5.0, about greater than 3.0 to 4.5, about 3.5 to 4.5, about 4.0 to 4.5, about greater than 3.0 to 4.0, about 3.5 to 4.0, or about greater than 3.0 to 3.5.

The size and hardness of valine crystals formed in the subsequent concentration step may be controlled by adjusting the pH of the fermentation broth. Specifically, when the pH of the fermentation broth is adjusted to the above-described range, followed by concentration, large and hard valine crystals can be formed, and accordingly, the moisture content of the separated valine crystals can be reduced. If the pH of the fermentation broth is outside the above-described range, the crystal size of valine crystals precipitated after concentration may be small, and it may be difficult to separate valine crystals from the mother liquor of fermentation broth. Additionally, since a relatively large amount of moisture is separated together with valine crystals, a large amount of energy is required for subsequent drying.

In the step of adjusting the pH of the fermentation broth, the pH adjustment may be performed using a conventional acidic substance or a conventional basic substance. However, the substance added to the fermentation broth for pH adjustment may be determined as long as it does not affect the physical properties or chemical structure of valine comprised in the fermentation broth. Additionally, considering that valine products will be used in foods or feeds, an edible acidic substance or an edible basic substance may be added.

After the above-described pH adjustment, a process such as heating the fermentation broth to completely dissolve valine that may be precipitated in the fermentation broth may be performed, if necessary. For example, the fermentation broth may be heated to about 60°C to about 90°C.

After the step of adjusting the pH of the fermentation broth is performed, a step of concentrating the fermentation broth to prepare a concentrated fermentation broth is performed.

In the concentration step, valine may be precipitated in the form of crystals by removing moisture from the fermentation broth. The concentration may be carried out in various ways. The concentration may be carried out using a conventional concentrator (e.g., paddle dryer, slurry drying equipment, reduced pressure concentrator, forced circulation concentrator, thin film concentrator, or rotary concentrator, *etc.)* according to the appropriate selection by those skilled in the art.

In the concentration step, the concentration may be performed by removing moisture in the fermentation broth at a rate of 60 g/L/hr to 150 g/L/hr (meaning that 60 g to 150 g of moisture per liter of fermentation broth is removed for 1 hour). In some cases, the concentration may be performed by removing moisture in the fermentation broth at a rate of 90 g/L/hr to 150 g/L/hr, 120 g/L/hr to 150 g/L/hr, 60 g/L/hr to 120 g/L/hr, 90 g/L/hr to 120 g/L/hr, or 60 g/L/hr to 90 g/L/hr. In the present disclosure, the concentration rate is a factor affecting the size of valine crystals. When the fermentation broth is concentrated at the above-described rate, relatively large-sized valine crystals are formed, and valine crystals may be easily separated from the mother liquor of fermentation broth. Accordingly, the moisture in valine crystals is reduced.

Subsequently, a separation step of separating valine crystals precipitated by concentration in the concentration step from the mother liquor is performed.

In the separation step, a solid-liquid separator such as a reduced-pressure membrane filtration device, a pressurized membrane filtration device, a centrifugal separator, etc. may be used to separate valine crystals comprising moisture from the concentrated fermentation broth. The above-described means are exemplary, and the means for performing the separation step are not limited to the above-described examples.

The mother liquor remaining after the separation of valine crystals may be used again for concentration. Accordingly, valine that did not form wet crystals or valine that precipitated as crystals of a certain size or less, which was not separated in the separation step of valine crystals and remained in the mother liquor may be recycled. Additionally, after circulation of the mother liquor, an additional process such as heating the fermentation broth to dissolve valine precipitated in the form of fine crystals again in the fermentation broth may be performed, if necessary.

According to the method for producing valine crystals according to one aspect of the present disclosure described above, valine crystals with a low moisture content, which can be easily separated, may be obtained by adjusting the pH of the fermentation broth comprising valine and concentrating the fermentation broth at a suitable rate.

The valine crystals produced according to one embodiment of the present disclosure may have a viscosity of greater than 0 cp to less than 80 cp. In some cases, the viscosity of the valine crystals may be greater than 10 cp to less than 80 cp, greater than 15 cp to less than 80 cp, greater than 20 cp to less than 80 cp, greater than 30 cp to less than 80 cp, greater than 40 cp to less than 80 cp, greater than 50 cp to less than 80 cp, greater than 60 cp to less than 80 cp, greater than 0 cp to less than 70 cp, greater than 10 cp to less than 70 cp, greater than 15 cp to less than 70 cp, greater than 20 cp to less than 70 cp, greater than 30 cp to less than 70 cp, greater than 40 cp to less than 70 cp, greater than 50 cp to less than 70 cp, greater than 60 cp to less than 70 cp, greater than 0 cp to less than 50 cp, greater than 10 cp to less than 50 cp, greater than 15 cp to less than 50 cp, greater than 20 cp to less than 50 cp, greater than 30 cp to less than 50 cp, greater than 40 cp to less than 50 cp, greater than 0 cp to less than 30 cp, greater than 10 cp to less than 30 cp, greater than 15 cp to less than 30 cp, greater than 20 cp to less than 30 cp, greater than 0 cp to less than 25 cp, greater than 10 cp to less than 25 cp, greater than 15 cp to less than 25 cp, greater than 20 cp to less than 25 cp, greater than 0 cp to less than 20 cp, greater than 10 cp to less than 20 cp, greater than 0 cp to less than 15 cp, greater than 10 cp to less than 15 cp, or greater than 0 cp to less than 10 cp. The valine crystals having a viscosity in the above-described range have a low moisture content and an appropriate size, and thus can be easily separated from the mother liquor.

The valine crystals produced according to one embodiment of the present disclosure may contain 10 wt% to 55 wt% of moisture. In some cases, the valine crystals may contain 20 wt% to 55 wt%, 30 wt% to 55 wt%, 40 wt% to 55 wt%, 50 wt% to 55 wt%, 10 wt% to 50 wt%, 20 wt% to 50 wt%, 30 wt% to 50 wt%, 40 wt% to 50 wt%, 10 wt% to 40 wt%, 20 wt% to 40 wt%, 30 wt% to 40 wt%, 10 wt% to 30 wt%, 20 wt% to 30 wt%, or 10 wt% to 20 wt% of moisture. Since the valine crystals having the moisture content in the above-described range have a relatively low moisture content, they can be used directly without a separate drying step or may be used only after slight drying, thereby reducing the energy required for the drying process.

The method for producing valine granules may comprise: preparing a fermentation broth comprising valine; adjusting the pH of the fermentation broth; concentrating the fermentation broth to prepare a concentrated fermentation broth; separating valine crystals comprising moisture from the concentrated fermentation broth; mixing the valine crystals with a seed to produce mixed granules of valine; and drying the mixed granules of valine.

In particular, the step of preparing a fermentation broth, the step of adjusting the pH of the fermentation broth, the step of concentrating the fermentation broth, and the step of separating valine crystals may be performed in the same manner as in the method for producing valine crystals previously described. Accordingly, in order to avoid duplication of explanation, the following description focuses on the step of mixing the valine crystals with a seed to produce mixed granules; and the step of drying the mixed granules.

The valine crystals obtained in the step of separating valine crystals are mixed with a seed in the step of preparing mixed granules to prepare valine granules.

The seed used in the step of preparing mixed granules, which is also called a crystal of a seed or a seed crystal, refers to a material used as a catalyst for crystallization or granulation of a liquid. Specifically, the seed in the present disclosure may refer to a crystal of valine. When the seed comes into contact with the fermentation broth, the solid components present in the fermentation broth are combined with the seed to form an aggregation, thereby forming granules.

The seed used in the step of preparing mixed granules may have an average particle size of 150 to 300 µm. Specifically, a seed having an average particle size of 150 to 250 µm, 200 to 300 µm, or 200 to 250 µm may be used, but is not limited thereto. The particle size of the seed used may ultimately affect the productivity of the granule preparation according to the present disclosure, and thus can be appropriately selected by those skilled in the art considering the desired moisture content, *etc.*

In the step of preparing mixed granules, a mixer granulator may be used. The granules may be obtained via the mixer granulator by injecting the seed at a constant rate through a feeder into the mixer granulator and simultaneously supplying the valine crystals obtained in the previous separation step. The mixer granulator may include a feeder for supplying a seed into the interior and a member for supplying valine crystals. Additionally, a gas nozzle for generating an airflow inside the chamber, a paddle for mixing the seed and valine crystals provided in the chamber, *etc.* may be provided inside the mixer granulator. The seed and valine crystals introduced into the chamber may clump together to form amino acid mixed granules.

According to one aspect of the present disclosure, amino acid mixed granules having a low moisture content and a large crystal size may be produced by mixing the seed and amino acid wet crystals. As previously discussed, according to the present disclosure, valine crystals having a large crystal size are precipitated, and since the valine crystals having a large crystal size may be easily separated from the fermentation broth, the moisture content in the valine crystals is relatively low. Additionally, as the valine crystals having a low moisture content and a seed are granulated in the mixer granulator in the step of preparing mixed granules, the thus-produced mixed granules of valine may also have a low moisture content and a large size.

As used herein, "granules" are macroscopic particles, which are larger-sized permanent aggregates composed of small particles such as powder, and may be particles with an average particle diameter of 50 µm to 5 mm, 75 µm to 4 mm, or 100 µm to 3 mm.

The mixed granules of valine obtained in the step of preparing mixed granules may contain 10 wt% to 50 wt% of moisture. In some cases, the mixed granules of valine may contain 20 wt% to 50 wt%, 30 wt% to 50 wt%, 40 wt% to 50 wt%, 10 wt% to 40 wt%, 20 wt% to 40 wt%, 30 wt% to 40 wt%, 10 wt% to 30 wt%, 20 wt% to 30 wt%, or 10 wt% to 20 wt% of moisture. This is a lower moisture content compared to that of amino acid granules obtained according to the prior art. Therefore, the amount of energy required to dry mixed granules of valine obtained according to the present disclosure may be reduced.

Subsequently, a drying step of drying the mixed granules of valine to obtain an amino acid product is performed.

In the drying step, there is no limitation on the drying method for drying the previously obtained mixed granules of valine. After drying, a valine product can be obtained. As used herein, the term "valine product" refers to a product in which valine contained in a fermentation broth is commercialized into various formulations. For example, the valine product may mean a valine-containing mixture in the form of granules. However, if necessary, the formulation of the valine product may be altered without changing the spirit of the present disclosure. Additionally, as described above, a subsequent process may be further performed to implement various formulations of the valine product. The above-described valine product may be used as an additive for animal feeds, *etc.,* and there is no limitation on the use thereof.

According to the present disclosure, amino acid mixed granules having a large size and a low moisture content may be produced by concentrating the fermentation broth containing amino acids to separate the amino acid wet crystals, followed by mixing the separated amino acid wet crystals with a seed. The amino acid mixed granules having a low moisture content have the advantage that less amount of energy is required for drying. In particular, according to the present disclosure, the moisture content in the amino acid mixed granules may be further reduced by adjusting the pH of the fermentation broth or controlling the concentration rate.

### [Mode for Carrying Out the Invention]

Hereinafter, the present disclosure will be described in detail by way of Examples. However, these Examples are merely preferred Examples given for illustrative purposes, and thus, the scope of the invention is not intended to be limited to or by these Examples. Meanwhile, technical features that are not described herein can be sufficiently understood and easily carried out by those skilled in the art in the technical field of the present disclosure or in a similar technical field.

### Experimental Example 1. Production of Valine Fermentation Broth via Cultivation of Microorganism of the genus Corynebacterium

In this Experimental Example, in order to produce granules containing L-valine, *Corynebacterium glutamicum* KCCM11338P (US 10072278 B2), one of the L-valine-producing strains, was cultured in a medium containing 5% glucose, 2% ammonium sulfate, 0.1% monopotassium phosphate, 0.05% MgSO₄·7H₂O, 2.0% corn steep liquor (CSL), 200 µg/L biotin and pH 7.2 at about 30°C for 72 hours to obtain a fermentation broth having the following composition. The fermentation broth contained the cultured medium and microorganisms, and the moisture content and composition were analyzed therefrom. Table 2 shows the results of the analysis of the composition of the valine fermentation broth.

**[Table 1]**

| **Composition** | **Value (g/L)** |
|---|---|
| Valine | 80.0 |
| Other amino acids | 8.8 |
| Organic acids | 0.7 |
| Inorganic substances | 11.0 |
| Moisture (%) | 89.0 |

### Experimental Example 2. Comparison Analysis of Moisture Contents in Amino Acid Wet Crystals According to pH of Fermentation broth

The fermentation broth with an L-valine concentration of 80 g/L prepared in Experimental Example 1 was divided into 2 L portions, and sulfuric acid was added thereto to prepare process solutions according to pH (7.5-3.0). In order to precipitate valine crystals from the prepared process solutions, a reduced pressure concentrator was used to concentrate the solutions at a rate of 60 g/L/hr at a vacuum pressure of 120 torr until the valine concentration of the process solutions became 200 g/L. The concentrated crystal slurry containing valine crystals was separated into solid and liquid using a basket separator to obtain L-valine crystals and mother liquor.

The moisture contents in the valine crystals according to the pH of the fermentation broth were compared by LOD analysis.

**[Table 2]**

| **pH of Fermentation broth** | **7.0** | **6.0** | **5.0** | **4.0** | **3.0** |
|---|---|---|---|---|---|
| Viscosity of slurry (cp) | 89 | 80 | 20.5 | 16.0 | 90 |
| Moisture content in crystals (wt%) | 45 | 40 | 32 | 30 | 39 |

The results of LOD moisture measurement showed that the moisture content in the valine crystals was low when the pH of the fermentation broth was greater than 3.0 to less than 6.0. Additionally, when the viscosity of the concentrated slurry was analyzed, it was confirmed that the viscosity of the concentrated slurry was low when the pH of the fermentation broth was greater than 3.0 to less than 6.0. The low viscosity of the concentrated slurry means that the size of the valine crystals contained in the concentrated slurry was large, and not many fine crystals were formed. In contrast, in the case of fermentation broth with pH 7.0, where the viscosity of the concentrated slurry was high, it was analyzed that many fine valine crystals were formed, resulting in a high slurry viscosity. Therefore, it was confirmed that the pH of the fermentation broth had an effect on the formation of valine crystals. Specifically, it was confirmed that large valine crystals were formed in a specific pH range, which resulted in a low viscosity of the concentrated slurry and low moisture in the separated crystals.

### Experimental Example 3. Comparison Analysis of Moisture Contents in Valine Crystals According to Concentration Rate of Fermentation broth (g/L/hr)

The fermentation broth with an L-valine concentration of 80 g/L prepared in Experimental Example 1 was divided into 5 L portions, and sulfuric acid was added thereto to adjust the pH of the fermentation broth to 3.8. In order to precipitate valine crystals, the solution was concentrated at a vacuum pressure of 120 torr using a vacuum evaporator until the concentration became 200 g/L. At this time, the concentration rate was varied from 18 g/L/hr to 120 g/L/hr for each batch, and the properties of the concentrated crystal slurry obtained according to the concentration rate were compared. The concentration rate was calculated as the concentration of concentrated valine per hour (g/L) based on the time taken for the concentration of valine to become 200 g/L when moisture was removed from the initial valine concentration of 80 g/L of the fermentation broth. The volume of condensate and the time taken for condensation were measured to calculate the concentration rate.

Additionally, the concentrated crystal slurry containingvaline crystals was separated into solid and liquid using a basket separator to obtain L-valine crystals and mother liquor. The moisture contents in valine crystals according to the concentration rate were compared. The moisture contents in valine crystals according to the concentration rate of the fermentation broth were compared by LOD analysis.

**[Table 3]**

| | | | | | |
|---|---|---|---|---|---|
| **Concentration Rate (g/L/hr)** | **18** | **30** | **60** | **90** | **120** |

| Viscosity of slurry (cp) | Measure ment not available | 99.7 | 16.0 | 8.3 | 6.7 |
|---|---|---|---|---|---|
| Moisture content in crystals (wt%) | Not separate d | 58 | 35 | 29 | 28 |

The results of LOD moisture measurement showed that as the concentration rate increased, the moisture content in the crystals decreased, confirming that the concentration rate affected the formation of L-valine crystals. In contrast, when the concentration rate was 18 g/L/hr, the crystals were formed in a gel-like form, rendering flow and separation difficult.

### Experimental Example 4. Production of Valine Crystals from Valine Fermentation broth

The pH of the fermentation broth was adjusted to 3.8 by adding sulfuric acid to 10 L of the fermentation broth with an L-valine concentration of 80 g/L. In order to precipitate valine crystals, a reduced pressure concentrator was used to concentrate the solution at a rate of 60 g/L/hr at a vacuum pressure of 120 torr until the concentration of the fermentation broth became 200 g/L.

The concentrated crystal slurry was separated into solid and liquid using a decanter to obtain 840 g of L-valine solids and 2850 mL of supernatant. At this time, the moisture content of the valine crystals was confirmed to be 37 wt%, and the valine crystal yield was 80.0% based on the fermentation broth.

### Experimental Example 5. Production of Valine Granules from Separated Crystals

In the same manner as in Experimental Example 4, valine crystals were produced, and the separated valine crystals and a pre-dried valine seed (valine content 75 w/w%) were mixed to produce crystals having a moisture content of 15% to 47%, and then valine mixed granule crystals were produced using a mixer granulator.

It was confirmed that mixed granules could be produced using a mixer granulator for all crystals having a moisture content of 15% to 47%.

**[Table 4]**

| **Mixed moisture (%)** | | **15.0** | **24.0** | **30.0** | **35.0** | **47.0** |
|---|---|---|---|---|---|---|
| Equipment | Type | CVG Mixer | | | | |
| | rpm | 2000 | | | | |
| Presence or absence of mixed granules | | ○ | ○ | ○ | ○ | ○ |

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within the metes and bounds of the claims, or equivalents of such metes and bounds are therefore intended to be embraced by the claims.

## Claims

1. A method for producing valine crystals, comprising:
preparing a fermentation broth comprising valine;
adjusting the pH of the fermentation broth;
concentrating the fermentation broth to prepare a concentrated fermentation broth; and
separating valine crystals comprising moisture from the concentrated fermentation broth.

2. The method of claim 1, wherein the pH of the fermentation broth is adjusted to be greater than 3.0 to less than 6.0 in the step of adjusting the pH of the fermentation broth.

3. The method of claim 1, wherein the step of concentrating the fermentation broth to prepare a concentrated fermentation broth is carried out by removing moisture in the fermentation broth at a rate of 60 g/L/hr to 150 g/L/hr.

4. The method of claim 1, wherein the valine crystals have a viscosity of greater than 0 to less than 80 cp.

5. The method of claim 1, wherein the valine crystals comprise 10 wt% to 55 wt% of moisture.

6. A method for producing valine granules, comprising:
preparing a fermentation broth comprising valine;
adjusting the pH of the fermentation broth;
concentrating the fermentation broth to prepare a concentrated fermentation broth;
separating valine crystals comprising moisture from the concentrated fermentation broth;
mixing the valine crystals with a seed to produce mixed granules of valine; and
drying the mixed granules of valine.

7. The method of claim 6, wherein the mixed granules comprise 10 wt% to 50 wt% of moisture.
